# EUROPEAN PATENT SPECIFICATION

(11) **EP 0 322 494 B1**
(45) Date of publication and mention of the grant of the patent: **07.04.1993**
(21) Application number: 87830472.4
(22) Date of filing: 29.12.1987
(51) Int. Cl.: C07C 209/82, C07C 211/52

(54) **A method for the treatment of dinitroanilines to reduce their nitrosamine content and/or to stabilise them against the formation of nitrosamines**
Methode zur Behandlung von Dinitroanilinen, um ihren Nitrosamingehalt zu vermindern und/oder zu deren Stabilisierung gegen die Bildung von Nitrosaminen
Méthode de traitement de dinitroanilines afin de réduire le taux de nitrosamine et/ou de les stabiliser contre la formation de nitrosamines

(43) Date of publication of application: 05.07.1989
(73) Proprietor: FINCHIMICA S.p.A., I-25025 Manerbio BS (IT)
(72) Inventor: Negri, Luigi, I-25036 Palazzolo Sull'Oglio (Brescia) (IT)
(74) Representative: Rambelli, Paolo

(56) References cited:
- EP-A- 0 151 725
- FR-A- 2 401 904
- US-A- 3 675 445

## Description

The present invention relates to a method for the treatment of nitroanilines to reduce their nitrosamine content and/or to stabilise them against the formation of nitrosamines.

The method relates in particular to the treatment of 2, 6-dinitro-N, N-di-n-propyl-4-trifluoromethyl aniline, known under the trade name of trifluralin , a compound which is widely used in agriculture as a herbicide.

The presence of a nitrosamine content greater than 0.5 ppm in dinitroaniline compositions used as herbicides is considered undesirable due to the confirmed carcinogenic properties of nitrosamines in animals.

The methods described in the technical literature for the removal of nitrosamines from crude trifluralin obtained by the synthesis method, include the treatment of the trifluralin with a concentrated aqueous solution of hydrochloric acid, or with hydrogen chloride gas (US patent No. 4,226,789) or with chlorides of inorganic acids (US patent No. 4,185,035) or with chlorine or bromine (US Patent No. 4,127,610).

It has, however, been found that even after these treatments, the di-n-propyl nitrosamine content increases during prolonged storage of the treated technical trifluralin and during subsequent industrial fusion and formulation operations for the preparation of herbicidal compositions. In particular, the increase in the nitrosamine content is encouraged by the presence of iron and is thus inevitable when trifluralin is stored in iron containers, even if they are lacquered.

Tests carried out by the Applicant have shown a progressive increase with time in the nitrosamine content of samples of technical trifluralin stored in glass containers and in glass and iron containers, subjected to heating to a temperature of 95°C.

A further confirmation of the problem of the reformation of nitrosamines following heating of purified trifluralin , is provided by the comparative examples in European patent application No. EP-A-126591 which relates to a method for the stabilisation of dinitroaniline compositions by the incorporation in the dinitroaniline of an addition compound of a bisulphite of ammonia or of an alkali metal and an aldehyde or a ketone.

US Patent No. 4,537,992 describes a method for the removal of the nitrosamines by subjection of the dinitroanilines to a treatment with acyl chlorides and aminobenzoates.

This method does not, however, provide any guarantee with regard to the stabilisation of the treated dinitroanilines against the reformation of nitrosamines.

German patent application No. DE-OS-333 45 157 describes a method for the production of dinitroanilines substantially free from nitrosamines and their stabilisation against the formation of nitrosamines.

The method provides for the treatment of the dinitroanilines with aromatic or aliphatic sulphonic acids, with chlorosulphonic acid or with amidosulphonic acid.

Amongst the compounds indicated, only dodecylbenzene sulphonic acid is used commercially, this, however, being used exclusively for the stabilisation of dinitroanilines which have previously been purified of nitrosamines. In fact, since this compound is a surfactant, its presence in the higher concentration required for the purification of crude nitroaniline may give rise to the undesired formation of foams and emulsions when water is present, even in small quantities, in the reactor used for the treatment.

The object of the present invention is to provide a method which constitutes an overall solution both to the problem of the removal of nitrosamines from crude trifluralin and to the problem of stabilising the purified trifluralin against the formation of nitrosamines.

For this purpose, the subject of the present invention is a method for the treatment of dinitroanilines having herbicidal action to reduce their nitrosamine content and/or to stabilise them against the formation of nitrosamines, characterised in that it comprises the steps of adding to the dinitroanilines an effective quantity of an aliphatic or aromatic chlorosulphonic acid having the general formula 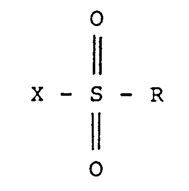
in which X is a halogen and R is selected from the group consisting of linear or branched aliphatic radicals having from 1 to 10 carbon atoms, cycloaliphatic C₅-C₆ radicals, aryl radicals and aryl radicals substituted with lower (C₁-C₃) alkyl groups.

Although the method is generally effective as regards the stabilisation and/or purification of dinitroanilines having herbicidal action, particular reference will be made to trifluralin in the following part of the present description.

In the method according to the invention, typically the above-mentioned sulphonyl chloride is added to the trifluralin in the fused state, in quantities of from 0.01 to 1% by weight with respect to the weight of the trifluralin . The stabilisation is typically carried out at a temperature higher than the melting point of the trifluralin and generally within the range 70 to 100°C. The sulphonyl chloride is preferably added, in this case, in a ratio of from 0.01 to 0.1% by weight with agitation and the agitation is continued for a period of between 1 and 4 hours. In the case of the purification treatment carried out on crude trifluralin , the treatment is effected at a temperature of between 95 and 110°C, the sulphonyl chloride being added in a ratio preferably of from 0.3 to 1% by weight. The agitation is continued for a period of between 8 and 10 hours.

The treatment according to the invention is effective even when precursor compounds for nitrosamines, such as, di-n-propylamines, for example, are present in the trifluralin subjected to treatment.

The results obtained by means of the method acccording to the invention will be illustrated by the following examples of experimental tests.

### Example 1 (Reference example)

The following example illustrates the phenomenon of the formation of nitrosamines in purified trifluralin satisfying the requirements established by the EPA, that is, having a nitrosamine content of less than 0.5ppm following heating in glass containers and in the presence of glass and iron.

120g of trifluralin , having a nitrosamine content (NO-DIPA below) of less than 0.5ppm, obtained by means of a purification treatment carried out in the laboratory with hydrochloric acid, were placed in a glass container into which a 10cm² test piece of sheet iron was introduced. The container was then kept at a temperature of 95°C for a period of from 12 to 150 hours. A parallel control test was carried out with the use of the same quantity of trifluralin without the sheet iron test piece.

Table I below indicates the nitrosamine contents after 50, 100 and 150 hours under the above conditions, the analyses being effected with a thermal energy analyser (TEA).

**TABLE I**

| CONTAINER | NO-DIPA content after 50 hours (ppm) | NO-DIPA content after 100 hours (ppm) | NO-DIPA content after 150 hours (ppm) |
|---|---|---|---|
| GLASS | 0.65 | 1.04 | 1.2 |
| GLASS + IRON | 1 | 2 | 2.3 |

The results in Table I confirm that the purification treatment with aqueous hydrochloric acid is not effective in stabilising the trifluralin and that the increase in the nitrosamine content is greater when iron is present in the container.

### Example 2

Various series of technical trifluralin samples were prepared containing known quantities of di-n-propyl nitrosamine (NO-DIPA). Some of the samples prepared also contained a known quantity of di-n-propylamine (DIPA).

The compositions of the prepared samples subjected to treatment are shown in Table II below.

**TABLE II**

| SERIES | NO-DIPA content (ppm) | DIPA content (ppm) |
|---|---|---|
| A | 0.24 | - |
| B | 1.93 | - |
| C | 3.5 | - |
| D | 0.24 | 2 |
| E | 0.24 | 10 |
| F | 1.97 | 2 |
| G | 1.97 | 10 |
| H | 2.5 | - |
| I | 9.2 | - |

120g of technical trifluralin were placed in a glass container into which a 10 cm² test piece of sheet iron was introduced and to which a known quantity of a chlorosulphonic aliphatic or aromatic acid selected from the above-mentioned group was added. The container was then kept at a temperature of 95°C for a period of from 12 to 160 hours.

A parallel control test was carried out for each sample, with the use of the same quantity of trifluralin but without the addition of the treatment additive. Samples were taken after 12, 48, 96, 150 and 160 hours and, in each case, their NO-DIPA contents were determined by TEA.

The results of the tests are shown in the following tables.

| SERIES A | | quantity of NO-DIPA (ppm) at various times | | | |
|---|---|---|---|---|---|
| ADDITIVE | % | 12 h | 48 h | 96 h | 150 h |
| METHANE SULPHONYL CHLORIDE | 0.1 | 0.05 | 0.05 | 0 05 | A s. |
| OCTANE SULPHONYL CHLORIDE | 0.1 | 0.05 | 0.05 | 0.05 | Abs. |
| BENZENE SULPHONYL CHLORIDE | 0.1 | 0.05 | 0.05 | 0 05 | Abs. |
| CONTROL | | 0.87 | 1.41 | 1.23 | 1.41 |

| SERIES B | | quantity of NO-DIPA (ppm) at various times | | | |
|---|---|---|---|---|---|
| ADDITIVE | % | 12 h | 48 h | 96 h | 150 h |
| METHANE SULPHONYL CHLORIDE | 0.1 | 0.05 | 0.05 | 0.05 | Abs. |
| OCTANE SULPHONYL CHLORIDE | 0.1 | 0.17 | 0.05 | 0.05 | Abs. |
| BENZENE SULPHONYL CHLORIDE | 0.1 | 1.61 | 0.33 | 0.05 | Abs. |
| CONTROL | | 2.6 | 2.58 | 2.8 | 2.53 |

| SERIES C | | quantity of NO-DIPA (ppm) at various times | | | |
|---|---|---|---|---|---|
| ADDITIVE | % | 12 h | 48 h | 96 h | 150 h |
| METHANE SULPHONYL CHLORIDE | 0.1 | 0.38 | 0.05 | 0.05 | |
| OCTANE SULPHONYL CHLORIDE | 0.1 | 0.05 | 0.05 | 0.05 | |
| BENZENE SULPHONYL CHLORIDE | 0.1 | 0.7 | 0.05 | 0.05 | |
| CONTROL | | 3.84 | 4.87 | 5.52 | |

| SERIES D | | quantity of NO-DIPA (ppm) at various times | | | |
|---|---|---|---|---|---|
| ADDITIVE | % | 12 h | 48 h | 96 h | 150 h |
| ETHANE SULPHONYL CHLORIDE | 0.1 | 0.06 | 0.05 | 0.05 | |
| BENZENE SULPHONYL CHLORIDE | 0.1 | 0.107 | 0.05 | 0.05 | |
| CONTROL | | 1.23 | 2.1 | 1.92 | |

| SERIES E | | quantity of NO-DIPA (ppm) at various times | | | |
|---|---|---|---|---|---|
| ADDITIVE | % | 12 h | 48 h | 96 h | 150 h |
| ETHANE SULPHONYL CHLORIDE | 0.1 | 0.07 | 0.05 | 0.05 | |
| BENZENE SULPHONYL CHLORIDE | 0.1 | 0.83 | 0.05 | 0.05 | |
| CONTROL | | 1.3 | 1.82 | 2.91 | |

| SERIES F | | quantity of NO-DIPA (ppm) at various times | | | |
|---|---|---|---|---|---|
| ADDITIVE | % | 12 h | 48 h | 96 h | 150 h |
| OCTANE SULPHONYL CHLORIDE | 0.1 | 0.74 | 0.44 | 0.05 | |
| p.TOLUENE SULPHONYL CHLORIDE | 0.1 | 0.33 | 0.05 | 0.05 | |
| CONTROL | | 2.76 | 2.86 | 3.72 | |

| SERIES G | | quantity of NO-DIPA (ppm) at various times | | | |
|---|---|---|---|---|---|
| ADDITIVE | % | 12 h | 48 h | 96 h | 150 h |
| OCTANE SULPHONYL CHLORIDE | 0.1 | 0.05 | 0.05 | 0.05 | |
| p.TOLUENE SULPHONYL CHLORIDE | 0.1 | 2.17 | 2.1 | 1.43 | |
| CONTROL | | 2.54 | 3.81 | 4.15 | |

| SERIES H | | QUANTITY OF NO-DIPA AT VARIOUS TIMES | | | |
|---|---|---|---|---|---|
| ADDITIVE | % | 12h | 48h | 96h | 160h |
| METHANE SULPHONYL CHLORIDE | 0.02 | 2.2 | 1.68 | 1.37 | 0.14 |
| BENZENE SULPHONYL CHLORIDE | 0.02 | 1.56 | 0.64 | 0.24 | 0.15 |
| CONTROL | | 2.4 | 2.57 | 2.86 | 2.52 |

| SERIES I | | QUANTITY OF NO-DIPA AT VARIOUS TIMES | | | |
|---|---|---|---|---|---|
| ADDITIVE | % | 12h | 48h | 96h | 160h |
| METHANE SULPHONYL CHLORIDE | 0.02 | 5.72 | 0.29 | 0.24 | 0.22 |
| BENZENE SULPHONYL CHLORIDE | 0.02 | 8.12 | 4.17 | 3.15 | 1.4 |
| CONTROL | | 10.66 | 11.85 | 12.02 | 10.68 |

The experimental tests reported in the tables clearly indicate, not only the stabilisation of the nitrosamine content, but above all a progressive reduction thereof with time, even after prolonged heat treatment. The effectiveness of the sulphochlorides according to the invention in the stabilisation and reduction occurs both with purified trifuralin , that is having a nitrosamine content which satisfies the EPA standards, and with technical trifluralin having a nitrosamine content greater than 0.5ppm. The sulphochlorides according to the invention are, furthermore, effective when added in concentrations of only 0.02% by weight with respect to the trifluralin and even when di-n-propylamine (DIPA) which, as is known, is thought to be a precursor of nitrosamines, is present in the trifluralin . It follows, therefore, that the additives according to the invention are able to display their action both at the stage of commercial formulation of herbicidal compositions including trifluralin , and in the treatment of technical trifluralin obtained by synthesis.

### Example 3

### Destruction of nitrosodipropylamine in crude trifluralin with chlorides of sulphonic acids.

On the basis of the positive results obtained in the stabilisation tests, the possibility of using chlorides of sulphonic acids as nitrosamine destroyers was also checked. For this purpose, a series of tests was carried out with the use of samples of trifluralin having NO-DIPA contents in the range 60 to 350ppm and sulphonic acid chlorides in quantities of between 0.3 and 1% with respect to the crude trifluralin , at temperatures of between 95 and 110°C, for periods of 8-10 hours.

### Test 1.

500g of crude trifluralin , having an initial NO-DIPA content of 119ppm, were introduced into a 1 litre flask provided with an agitator, and to this were added 2.5g of methane sulphonylchloride, equal to 0.5% by weight of the crude trifluralin . The mixture was heated to 95°C for 8 hours with rapid agitation. A sample was then taken and analysed by TEA and a NO-DIPA content of 0.07ppm was found.

The following table summarises the conditions and results of the tests carried out.

| TEST | INITIAL NO-DIPA (ppm) | FINAL NO-DIPA (ppm) | R-SO₂Cl | %to trifluraline | TEMPERATURE (°C) | TIME (HOURS) |
|---|---|---|---|---|---|---|
| 1° | 350 | ABSENT | BSCl | 1 | 95 | 8 |
| 2° | 350 | ABSENT | MSCl | 1 | 95 | 8 |
| 3° | 110 | 0.06 | MSCl | 1 | 95 | 8 |
| 4° | 110 | 0.25 | OSCl | 1 | 95 | 8 |
| 5° | 350 | ABSENT | MSCl | 0.5 | 95 | 8 |
| 6° | 119 | 0.07 | MSCl | 0.5 | 95 | 8 |
| 7° | 60 | 0.15 | MSCl | 0.5 | 95 | 8 |
| 8° | 60 | 0.3 | BSCl | 0.5 | 105 | 8 |
| 9° | 101 | 0.18 | MSCl | 0.5 | 100 | 10 |
| 10° | 125 | 0.35 | OSCl | 0.5 | 100 | 10 |
| 11° | 125 | 0.15 | MSCl | 0.5 | 95 | 10 |
| 12° | 110 | 0.31 | BSCl | 0.3 | 105 | 10 |
| 13° | 60 | 0.28 | OSCl | 0.3 | 110 | 10 |
| 14° | 350 | 0.22 | MSCl | 0.3 | 95 | 8 |
| 15° | 138 | 0.18 | MSCl | 0.3 | 95 | 8 |
| BSCl BENZENE SULPHOCHLORIDE | | | | | | |
| OSCl OCTANE SULPHOCHLORIDE | | | | | | |
| MSCl METHANE SULPHOCHLORIDE | | | | | | |

In all the tests carried out, the term "absent" relates to the NO-DIPA content and should be understood to mean that the nitrosamine content was less than the lower threshold limit of the TEA instrument.

In all the tests carried out the final NO-DIPA content, after treatment for 8-10 hours, was less than the limit of 0.5ppm required by the standards.

## Claims

1. A method for the treatment of dinitroanilines to reduce their nitrosamine content and/or to stabilise them against the formation of nitrosamines, characterised in that it comprises the steps of adding to the dinitroaniline compositions an effective quantity of an aliphatic or aromatic halosulphonic acid having the general formula: 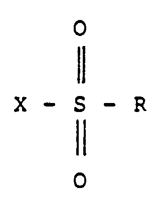 where X is a halogen and R is selected from the group consisting of aliphatic radicals having from 1 to 10 carbon atoms, cycloaliphatic C₅-C₆ radicals, aryl radicals and aryl radicals substituted with C₁-C₃ alkyl groups.

2. A method according to Claim 1, characterised in that a quantity of the halosulphonic acid of between 0.01 and 1% by weight of the dinitroaniline is added to the dinitroaniline in the fused state with agitation.

3. A method according to Claim 1, particularly for the stabilisation of technical-quality dinitroanilines against the formation of nitrosamines, characterised in that the halosulphonic acid is added to the technical dinitroaniline in quantities of from 0.01 to 0.1% by weight with respect to the dinitroaniline.

4. A method according to Claim 1, particularly for the destruction of nitrosamines in crude dinitroaniline, characterised in that the halosulphonic acid is added to the crude dinitroaniline at a temperature of from 95 to 110°C, in quantities of from 0.3 to 1% by weight with respect to the dinitroaniline.

5. A method according to any one of Claims 1 to 4, characterised in that the halosulphonic acid is selected from the group consisting of methane sulphonyl chloride, ethane sulphonyl chloride, octane sulphonyl chloride, benzene sulphonyl chloride, and toluene sulphonyl chloride.

6. A method according to any one of Claims 1 to 5 characterised in that the dinitroaniline is trifluralin .

## Patentansprüche

1. Verfahren zur Behandlung von Dinitroanilinen zur Reduzierung ihres Nitrosamingehaltes und/oder zu deren Stabilisierung gegenüber der Bildung von Nitrosaminen, dadurch gekennzeichnet, daß es das Hinzufügen einer wirksamen Menge einer aliphatischen oder aromatischen Halogensulfonsäure der allgemeinen Formel: 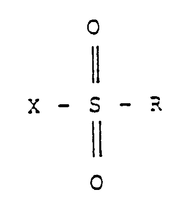 in der X ein Halogenatom ist und R aus der Gruppe aliphatische Reste mit 1 bis 10 Kohlenstoffatomen, cycloaliphatische C₅-C₆-Resten, Arylreste und mit C₁-C₃-Alkylresten substituierte Arylreste ausgewählt ist, zu der Dinitroanilinzusammensetzung umfaßt.

2. Verfahren gemäß Anspruch 1, dadurch gekennzeichnet, daß unter Rühren eine Menge der Halogensulfonsäure von 0,01 bis 1 Gew.-%, bezogen auf das Dinitroanilin, dem geschmolzenen Dinitroanilin zugefügt wird.

3. Verfahren gemäß Anspruch 1, insbesondere zur Stabilisierung von Dinitroanilinen in technischer Qualität gegenüber der Bildung von Nitrosaminen, dadurch gekennzeichnet, daß die Halogensulfonsäure zu dem technischen Dinitroanilin in Mengen von 0,01 bis 0,1 Gew.-%, bezogen auf das Dinitroanilin, hinzugefügt wird.

4. Verfahren gemäß Anspruch 1, insbesondere zur Zerstörung von Nitrosaminen in rohen Dinitroanilinen, dadurch gekennzeichnet, daß die Halogensulfonsäure zu dem rohen Dinitroanilin bei einer Temperatur von 95 bis 110°C in Mengen von 0,3 bis 1 Gew.-%, bezogen auf das Dinitroanilin, hinzugefügt wird.

5. Verfahren gemäß einem der Ansprüche 1 bis 4, dadurch gekennzeichnet, daß die Halogensulfonsäure aus der Gruppe Methansulfonsäurechlorid, Ethansulfonsäurechlorid, Octansulfonsäurechlorid, Phenylsulfonsäurechlorid und Toluolsulfonsäurechlorid ausgewählt ist.

6. Verfahren gemäß einem der Ansprüche 1 bis 5, dadurch gekennzeichnet, daß das Dinitroanilin 2,6-Dinitro-N,N-di-n-propyl-4-trifluormethylanilin (Trifluralin) ist.

## Revendications

1. Procédé pour le traitement des dinitroanilines en vue de réduire leur teneur en nitrosamine et/ou de les stabiliser contre la formation de nitrosamines, caractérisé en ce qu'il comporte les étapes d'ajouter aux compositions de dinitroanilines une quantité efficace d'un acide halosulfonique aromatique ou aliphatique ayant la formule générale 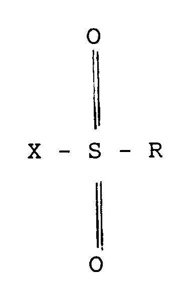 où X est un halogène et R est choisi à partir du groupe constitué des radicaux aliphatiques ayant de 1 à 10 atomes de carbone, radicaux C₅-C₆ cycloaliphatiques, radicaux aryles et radicaux aryle substitués avec des groupes alkyles C₁-C₃.

2. Procédé selon la revendication 1, caractérisé en ce qu'une quantité d'acide halosulfonique comprise entre 0,01 et 1% en poids de dinitroaniline est ajoutée à la dinitroaniline à l'état fondu avec agitation.

3. Procédé selon la revendication 1, particulièrement pour stabiliser des dinitroanilines de qualité industrielle contre la formation de nitrosamines, caractérisé en ce que de l'acide halosulfonique est ajouté à la dinitroaniline industrielle dans des quantités allant de 0,01 à 0,1% en poids par rapport à la dinitroaniline.

4. Procédé selon la revendication 1, particulièrement pour la décomposition des nitrosamines dans de la dinitroaniline brute, caractérisé en ce que de l'acide halosulfonique est ajouté à la dinitroaniline brute à une température allant de 95 à 110°C, dans des quantités allant de 0,3 à 1% en poids par rapport à la dinitroaniline.

5. Procédé selon l'une quelconque des revendications 1 à 4, caractérisé en ce que l'acide halosulfonique est choisi parmi le groupe constitué du chlorure de méthane sulfonyle, chlorure d'éthane sulfonyle, chlorure d'octane sulfonyle, chlorure de benzène sulfonyle et chlorure de toluène sulfonyle.

6. Procédé selon l'une quelconque des revendications 1 à 5 caractérisé en ce que la dinitroaniline est la trifluraline.
